(19) Europäisches Patentamt
European Patent Office
Office européen des brevets

(11) Veröffentlichungsnummer: **0 245 729**
**A2**

# (12) EUROPÄISCHE PATENTANMELDUNG

(21) Anmeldenummer: **87106379.8**

(22) Anmeldetag: **30.04.87**

(51) Int. Cl.$^3$: **C 07 D 209/48**
C 08 G 75/30

(30) Priorität: **13.05.86 DE 3616066**

(43) Veröffentlichungstag der Anmeldung:
**19.11.87 Patentblatt 87/47**

(84) Benannte Vertragsstaaten:
**DE FR GB IT NL**

(71) Anmelder: **BAYER AG**
**Konzernverwaltung RP Patentabteilung**
**D-5090 Leverkusen 1 Bayerwerk(DE)**

(72) Erfinder: **Reuter, Knud, Dr.**
**Scheiblerstrasse 99**
**D-4150 Krefeld(DE)**

(54) **Aromatische (Poly)-Sulfonimide und Verfahren zu ihrer Herstellung.**

(57) Die Erfindung betrifft ein Verfahren zur Herstellung aromatischer (Poly)-Sulfonimide und neue aromatische (Poly)-Sulfonimide.

EP 0 245 729 A2

Croydon Printing Company Ltd.

0245729

BAYER AKTIENGESELLSCHAFT 5090 Leverkusen, Bayerwerk
Konzernverwaltung RP
Patentabteilung Eck/Ke-c

# Aromatische (Poly)-Sulfonimide und Verfahren zu ihrer Herstellung

Die Erfindung betrifft ein Verfahren zur Herstellung aromatischer (Poly)-Sulfonimide und neue aromatische (Poly)-Sulfonimide.

Die Herstellung aromatischer Sulfone ist bekannt, siehe z.B. Ullmanns Encyclopädie der Technischen Chemie, 4. Auflage, Bd. 22, S. 323, z.B. Oxidation von Sulfiden bzw. Sulfoxiden und Sulfonierung aromatischer Kohlenwasserstoffe, Umlagerung von Sulfonsäureestern zu Hydroxydiphenylsulfonen, die Addition von Sulfinsäuren an Chinone und Chinonimine sowie die nucleophile Substitution von Chlor in o-oder p-Chlornitrobenzolen.

Die Herstellung von Sulfonen aus Nitroaromaten durch Substitution einer $NO_2$-Gruppe ist ebenfalls bekannt, z.B. die Synthese von Poly(arylsulfonyl)benzolen durch Umsetzung von Chlornitrobenzolen mit Arylsulfinaten (J. Chem. Soc. 1936, 216, 1937, 246 und 1939, 902).

Bekanntlich reicht eine zweite Nitrogruppe in o- oder p-Stellung aus, um den Austausch einer $NO_2$-Gruppe gegen Sulfinat in guter Ausbeute zu ermöglichen (J. Org. Chem. 41 (9), 1560 (1976), C.A. 87, 565 (1977)).

Es wurden nun neue (Poly)Arylsulfonimide und ein neues Verfahren zur Herstellung durch nucleophile aromatische Substitution von Nitrogruppen gefunden.

Die neuen aromatischen Sulfone entsprechen der Formel (I)

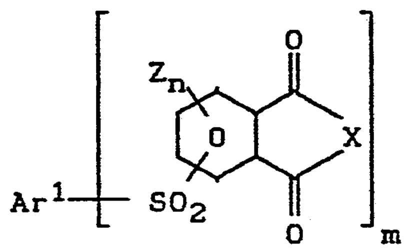

(I),

in welcher

Z für $C_1$-$C_4$-Alkyl, $C_6$-$C_{10}$-Aryl, $C_1$-$C_4$-Alkoxy $C_6$-$C_{10}$ Aryloxy, $C_1$-$C_4$-Dialkylamino, $C_6$-$C_{10}$-Aryl-$C_1$-$C_4$ alkylamino, $C_6$-$C_{10}$-diarylamino steht,

n für die Zahl 0,1,2 oder 3

m für die Zahl 1 oder 2 steht,

X für O oder N-R" und

R" für $C_1$-$C_{18}$ Alkyl(en), $C_5$-$C_{10}$-Cycloalkyl(en) oder $C_6$-$C_{14}$-Aryl(en) steht und

$Ar^1$ für gegebenenfalls mit $C_1$-$C_4$-Alkyl, oder Halogen wie Cl, Br oder $NO_2$ substituiertes $C_6$-$C_{14}$-Aryl (m=1), oder $C_6$-$C_{14}$-Arylen (m=2) steht,

wobei

$Ar^1$ auch vom Diphenylether (-Ar-O-Ar-) oder Diphenylsulfon (-Ar-$SO_2$-Ar-) abgeleitet sein kann, wobei die Ar unabhängig voneinander für $C_6$-Arylenreste, vorzugsweise -1,4- und -1,3-Phenylenreste stehen.

Durch Verseifung können aus den Sulfonen der Formel (I) die entsprechenden Carbonsäuren der Formel (Ia)

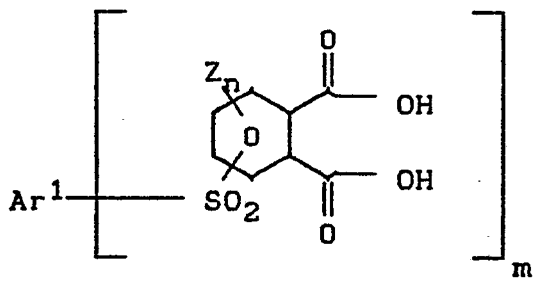

(Ia),

worin $Ar^1$, $Z_n$ und m die bei Formel (I) angegebene Bedeutung haben, hergestellt werden. Die Verseifung kann für den Fall, daß X in Formel (I) für N-R" steht (wobei R" die bei Formel (I) angegebene Bedeutung hat) in besonderen Fällen so durchgeführt werden, daß gegebenenfalls die Amidsäuren isoliert werden können. Amidsäuren können auch

erhalten werden, wenn Anhydride der Formel (I) (für den Fall, daß in Formel (I) X = O ist) mit Ammoniak oder primären oder sekundären Aminen umgesetzt werden.

Ein weiterer Gegenstand der Erfindung sind die neuen aromatischen (Poly)sulfonimide der Formel (II)

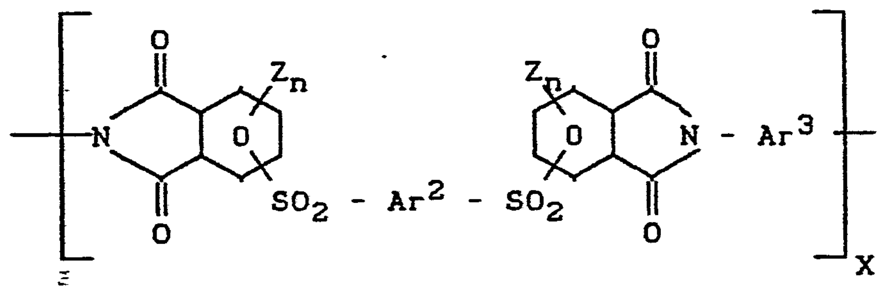
(II),

in welcher

Z und n die bei Formel I angegebene Bedeutung haben und

$Ar^2$ und $Ar^3$ unabhängig voneinander für $C_6$-$C_{14}$-Arylenreste stehen und

X für eine ganze Zahl von 2 bis 100, vorzugsweise 2 bis 50 steht.

Die Molgewichte $M_n$ betragen 1000 bis 100000, vorzugsweise 1000 bis 50000.

Ein weiterer Gegenstand der Erfindung ist ein Verfahren zur Herstellung der Sulfonimide der Formel (I), dadurch gekennzeichnet, daß Arylsulfinsäuren oder deren Salze der Formel (III)

$$Ar^1\left[SO_2M\right]_m \qquad (III),$$

in welcher

$Ar^1$ und m die bei Formel I angegebene Bedeutung haben und

M für Wasserstoff oder Alkali wie Li, Na, K und die $NR_4$-Gruppe steht, in welcher R für $C_1$-$C_4$-Alkyl steht,

mit aromatischen, Nitrogruppen-haltigen Imiden der Formel (IV)

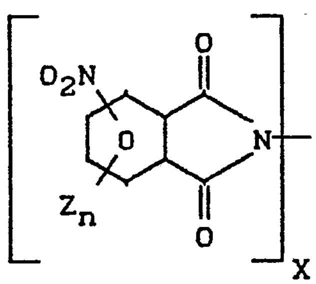

(IV),

in welcher

R", Z, n und X die in Formel (I) angegebene Bedeutung haben,

umgesetzt werden.

Als Lösungsmittel der Umsetzung von Arylsulfinsäuren der Formel (III) mit den Imiden der Formel (IV) können übliche dipolare, aprotische Lösungsmittel wie Dimethylsulfoxid, Dimethylformamid, Hexamethylphosphorsäuretriamid u.s.w. verwendet werden. Die Umsetzung kann bei einer Temperatur von 60 bis 160° C, gegebenenfalls bei einem Überdruck bis zu 10 bar durchgeführt werden.

Sollen Anhydride der Formel (I), (X=O in Formel I) hergestellt werden, so können die Imide der Formel I (X = N-R" in Formel I) zu den Carbonsäuren der Formel Ia auf übliche Weise verseift werden. Diese können dann auf übliche Weise, z.B. durch Umsetzung mit geeigneten Anhydriden, z.B. Essigsäureanhydrid, oder durch Erhitzen in Substanz auf Temperaturen bis ca. 300° C, zu den Anhydriden umgesetzt werden.

Die neuen aromatischen (Poly)sulfonimide der Formel (II) können durch Umsetzung von nitrogruppenhaltigen Imiden der Formel (IV), in welcher dann X für die Zahl 2 stehen muß und R" für $C_1$-$C_{18}$ Alkylen, $C_5$-$C_{10}$ Cycloalkylen oder $C_6$-$C_{14}$ Arylen stehen muß, mit Arylsulfinsäuren der Formel (III), in welcher dann m für die Zahl 2 stehen muß und $Ar^1$ für $C_6$-$C_{14}$-Arylen oder Diphenylether(-ArOAr-) oder Diphenylsulfon ($-ArSO_2Ar-$) stehen muß, in den oben genannten Lösungsmitteln bei den oben genannten Temperaturen umgesetzt werden.

Erfindungsgemäß einzusetzenden Arylsulfinsäuren der Formel III sind z.B. Benzolsulfinsäure, p-Toluolsulfinsäure, p-Chlorbenzolsulfinsäure, p-Nitro-Benzolsulfinsäure, Benzol-1,3-disulfinsäure, Toluol-disulfinsäure, Diphenylether-4,4'-disulfinsäure, Diphenylsulfon-3,3'-disulfinsäure usw.

Diese können z.B. umgesetzt werden z.B. mit 3-Nitro-N-methylphthalimid, 4-Nitro-N-methylphtalimid, 4-Nitro-N-cyclohexylphthalimid, den Imiden der Formeln (VIII) und (IX) usw.

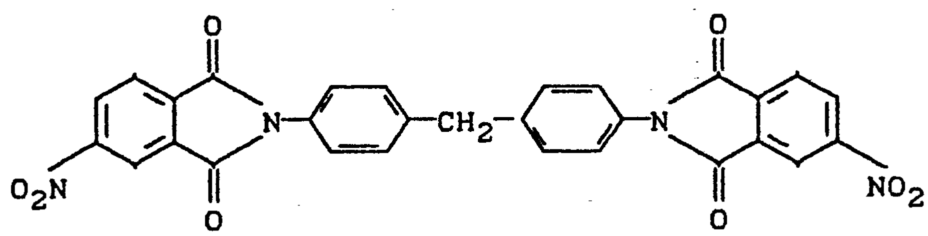

(VIII),

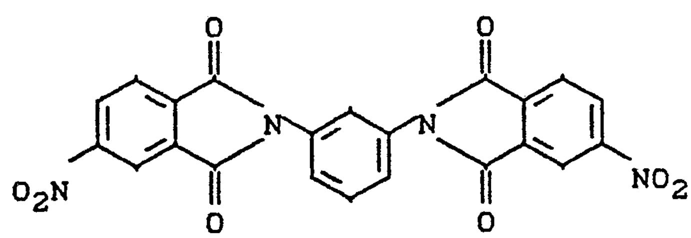

(IX).

Die nach dem erfindungsgemäßen Verfahren hergestellten Produkte eignen sich z.B. als Rohstoffe für Kunststoffe, beispielsweise derart, daß hochmolekulare Produkte zu thermoplastischen Werkstoffen verarbeitet werden, oder derart, daß niedermolekulare Produkte, welche geeignete funktionelle Gruppen tragen (z.B. Cl, $NO_2$, $NH_2$, cyclisches Anhydrid, Amid, Carboxyl usw.) mit dazu passenden Reaktionspartnern zu hochmolekularen Produkten umgesetzt und diese anschließend zu thermoplastischen Werkstoffen verarbeitet werden.

Beispiel 1

54,9 g Natriumbenzolsulfinat (70,2 %ig) werden in 300 ml DMSO und 200 ml Toluol azeotrop am Wasserabscheider entwässert. Für die Entfernung restlicher Spuren Wasser wird anschließend ein Soxhlet-Extraktor, der mit Molekularsieb 4 A gefüllt ist, aufgesetzt und 1 h darunter zum Rückfluß erhitzt. Danach wird eine Destillationsapparatur aufgesetzt und bis zu einer Innentemperatur von 145° C die Hauptmenge Toluol und wenig DMSO abdestilliert. Anschliessend werden 51,8 g 4-Nitro-N-methylphthalimid zugegeben und das Reaktionsgemisch 6 h auf 120° C erhitzt. Danach wird das Gemisch in viel Wasser gegeben und das ausgefällte Rohprodukt aus Essigsäure umkristallisiert. Reinausbeute 24,3 g (Schmelzpunkt 190° C), Struktur nach NMR- und IR-Spektrum sowie Elementaranalyse:

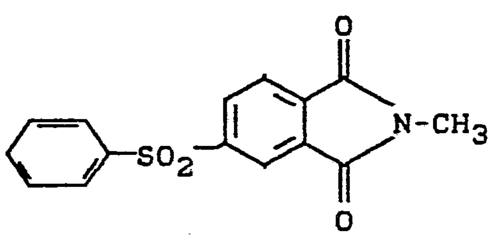

Beispiel 2

10,95 g Natrium-p-toluolsulfinat (76,7 %ig) in 60 ml DMSO und 40 ml Toluol werden nach dem Verfahren von Beispiel 1 entwässert. Nach Eindestillation bis zur Innentemperatur von ca. 145° C werden 10,35 g 4-Nitro-N-methylphthalimid zugegeben und das Reaktionsgemisch 6 h auf 150° C erhitzt. Danach wird in viel Wasser gegeben und mit Methylenchlorid extrahiert. Der nach Abdestillieren des Lsm. verbleibende Rückstand der organischen Phase wird mit Methanol gewaschen. Man erhält 6,6 g Rohprodukt, das aus Essigsäure umkristallisiert werden kann, Schmelzpunkt 185-187° C. Struktur nach NMR- und IR-Spektrum sowie Elementaranalyse:

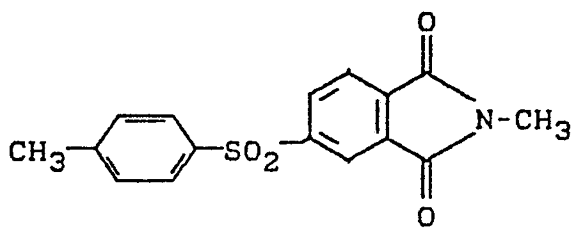

Beispiel 3

9,92 g Natrium-p-chlorbenzolsulfinat und 10,35 g 4-Nitro-N-methylphthalimid werden in 100 ml wasserfreiem DMSO 24 h bei 150° C umgesetzt. Bei Fällung des Reaktionsgemisches in Wasser werden 2,21 g eines unreinen Produktes erhalten, das nach Waschen mit Methanol durch HMR- und Massenspektrum sowie Elementaranalyse als im wesentlichen folgende Verbindung identifiziert wird:

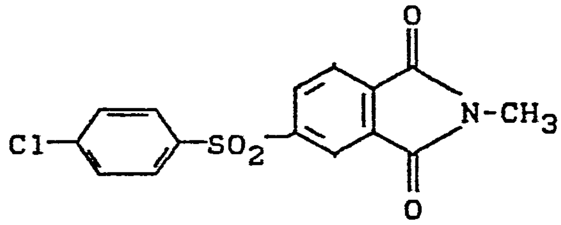

Beispiel 4

Entsprechend dem Verfahren von Beispiel 1 werden 10,95 g Natrium-p-toluolsulfinat in 120 ml DMSO und 100 ml Toluol entwässert. Nach Eindestillieren bis zur Innentemperatur von ca. 145° C werden 10,35 g 3-Nitro-N-methylphthalimid zugegeben und das Reaktionsgemisch 24 h auf 150° C erhitzt. Danach wird das Gemisch auf Eis gegeben und die Ausfällung mit Wasser gewaschen. Das Produkt (6,8 g) schmilzt bei 194-206° C und besteht nach NMR-Spektrum und Elementaranalyse aus folgender Verbindung:

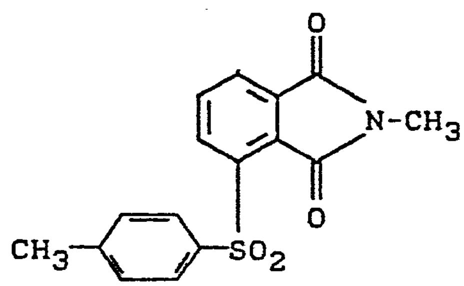

Beispiel 5

11,2 g Natrium-benzol-1,3-disulfinat werden entsprechend dem Verfahren in Beispiel 1 in 60 ml DMSO/40 ml Toluol entwässert. Nach Eindestillieren bis 145°C werden 18,4 g 4-Nitro-N-methylphthalimid (Molverhältnis 1:2) zugegeben und das Reaktionsgemisch 6 h auf 120°C erhitzt. Danach werden durch Eingießen in Methanol 3,6 g eines Rohproduktes gefällt, das mit Methanol gewaschen wird und nach NMR- und IR-Spektrum sowie Elementaranalyse zum großen Teil aus folgender Verbindung besteht:

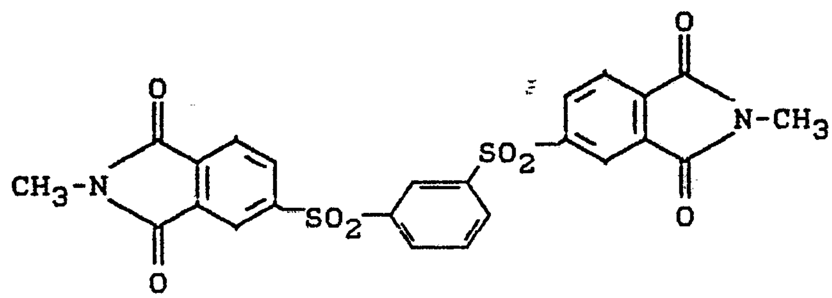

Beispiel 6

15,3 g Natrium-diphenylether-4,4'-disulfinat werden entsprechend Beispiel 5 mit 18,4 g 4-Nitro-N-methylphthalimid umgesetzt, wobei insgesamt 18 h auf 120-150°C erhitzt wird. Bei analoger Aufarbeitung wie in Beispiel 5 erhält man 4,5 g eines Rohproduktes, das nach NMR-Spektrum und Elementaranalyse einen großen Anteil folgender Verbindung enthält:

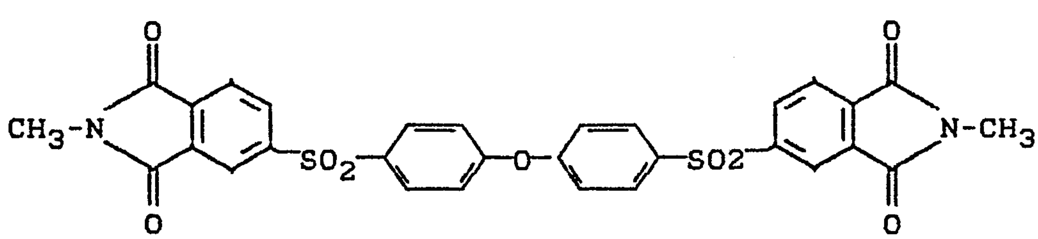

Beispiel 7

10,95 g Natrium-p-toluolsulfinat (76,7 %ig) und 10,35 g 4-Nitro-N-methylphthalimid werden in 100 ml wasserfreiem DMF 24 h zum Rückfluß erhitzt. Nach Abkühlen wird filtriert und die Mutterlauge in Wasser gegeben. Man erhält 4 g eines Rohproduktes, das an seienm Schmelzpunkt (187-189°C nach Umkristallisieren aus Essigsäure) und seinen spektroskopischen Daten als identisch mit dem Produkt des Beispiels 2 identifiziert wird (4-(p-Tolylsulfonyl)-N-methylphthalimid).

Beispiel 8

7 g des in Beispiel 2 bzw. 7 beschriebenen Produktes 4-(p-Tolylsulfonyl)-methylphthalimid werden mit 13,2 g 45 %iger wäßriger NaOH und 23 ml $H_2O$ hydrolysiert (24 h Rückfluß). Das Reaktionsgemisch wird dann mit Schwefelsäure auf pH 1 eingestellt und das ausgefallene Produkt abgesaugt und neutral gewaschen. Schmelzpunkt 140°C, die Elementaranalyse ist für folgende Verbindung korrekt:

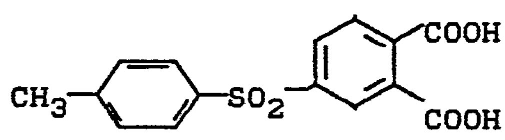

Das Produkt kann mit Essigsäureanhydrid in das cyclische Anhydrid umgewandelt werden.

Patentansprüche

1) Aromatische Sulfone der Formel (I)

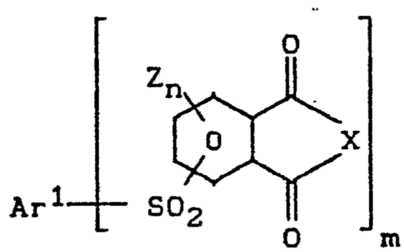

(I),

in welcher

Z für $C_1$-$C_4$ Alkyl, $C_6$-$C_{10}$-Aryl, $C_1$-$C_4$ Alkoxy, $C_6$-$C_{10}$ Aryloxy, $C_1$-$C_4$-Dialkylamino, $C_6$-$C_{10}$ Aryl-$C_1$-$C_4$ alkylamino, $C_6$-$C_{10}$-diarylamino steht,

n für die Zahl 0,1,2 oder 3,

m für die Zahl 1 oder 2 steht,

X für O der N-R" und

R" für $C_1$-$C_{18}$ Alkyl(en), $C_5$-$C_{10}$-Cycloalkyl(en) oder $C_6$-$C_{14}$-Aryl(en) steht und

$Ar^1$ für gegebenenfalls mit $C_1$-$C_4$ Alkyl oder Halogen wie Cl, Br oder $NO_2$ substituiertes $C_6$-$C_{14}$ Aryl (m=1) oder $C_6$-$C_{14}$ Arylen (m=2) steht,

wobei

$Ar^1$ auch von Diphenylether (-Ar-O-Ar-) oder Diphenylsulfon (-Ar-$SO_2$-Ar-) abgeleitet sein kann, wobei die Ar unabhängig voneinander für $C_6$-Arylenreste stehen.

2. Carbonsäuren der Formel Ia

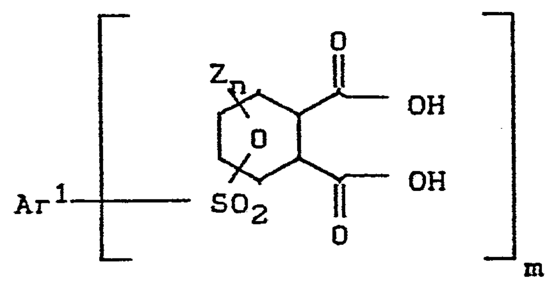

(Ia),

worin $Ar^1$, $Z_n$ und m die bei Formel (I) in Anspruch 1 angegebene Bedeutung haben.

3. Aromatische (Poly)sulfonimide der Formel II

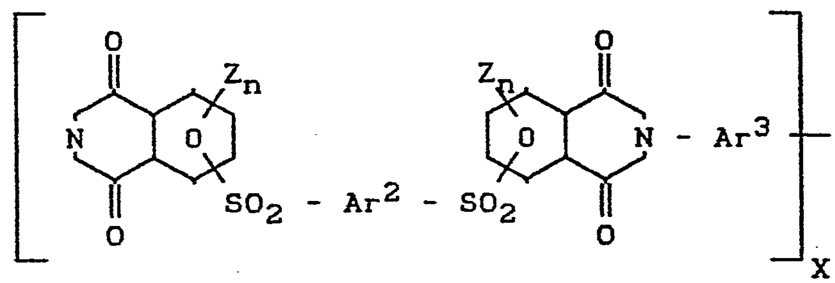

(II),

in welcher

Z und n die bei Formel I angegebene Bedeutung haben und

$Ar^2$ und $Ar^3$ unabhängig voneinander für $C_6$-$C_{14}$-Arylenreste stehen und

X für eine ganze Zahl von 2 bis 100 steht.

4. Verfahren zur Herstellung der Sulfonamide der Formel (I), dadurch gekennzeichnet, daß Arylsulfinsäuren oder deren Salze der Formel (III)

$$Ar^1\left[SO_2M\right]_m \qquad (III),$$

in welcher

$Ar^1$ und m die bei Formel I in Anspruch 1 angegebene Bedeutung haben und

M für Wasserstoff oder Alkali wie Li, Na, K und die $NR_4$-Gruppe steht, in welcher R für $C_1$-$C_4$ Alkyl steht,

mit aromatischen, Nitrogruppen-haltigen Imiden der Formel (IV)

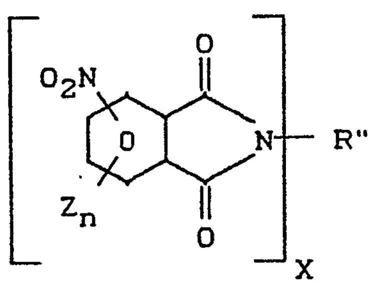

(IV),

in welcher

R", Z, n und X die in Formel (I) in Anspruch 1 angegebene Bedeutung haben, umgesetzt werden.